# EUROPÄISCHE PATENTSCHRIFT

(11) **EP 0 584 631 B1**
(45) Veröffentlichungstag und Bekanntmachung des Hinweises auf die Patenterteilung: **16.07.1997**
(21) Anmeldenummer: 93112778.1
(22) Anmeldetag: 10.08.1993
(51) Int. Cl.: C07D 307/32

(54) **Verfahren zur Herstellung von 3-(2'-Acyloxyethyl)-dihydro-2(3H)furanonen**
Process of preparing 3-(2'-acyloxyethyl)-dihydro-2(3H)furanones
Procédé de préparation de 3-(2'-acyloxyéthyl)-dihydro-2(3H)furanones

(30) Priorität: 28.08.1992 DE 4228668
(43) Veröffentlichungstag der Anmeldung: 02.03.1994
(73) Patentinhaber: BASF Aktiengesellschaft, 67063 Ludwigshafen (DE)
(72) Erfinder: Schnurr, Werner, Dr., D-6719 Herxheim (DE); Fischer, Rolf, Dr., D-6900 Heidelberg (DE); Goetz, Norbert, Dr., D-6520 Worms 1 (DE); Kuekenhoehner, Thomas, Dr., D-6737 Boehl-Iggelheim (DE)

(56) Entgegenhaltungen:
- EP-A- 0 246 581
- EP-A- 0 284 969
- DE-A- 1 493 211
- US-A- 4 734 521
- JOURNAL OF ORGANIC CHEMISTRY. Bd. 39, Nr. 25 , 1974 , EASTON US Seiten 3728 - 3730 B. GANEM ET AL. 'Ferric Chloride in Acetic Anhydride. A Mild and Versatile Reagent for the Cleavage of Ethers'
- JOURNAL OF ORGANIC CHEMISTRY, Bd. 39, Nr. 25, 1974, Easton, US, Seiten 3728-3730, B. GANEM et al.

## Beschreibung

Die vorliegende Erfindung betrifft ein Verfahren zur Herstellung von 3-(2'-Acyloxyethyl)-dihydro-2(3H)furanonen durch Umsetzung von 3-(2'-Oxyethyl)-dihydro-2(3H)furanonen mit Carbonsäuren, Carbonsäureanhydriden und Carbonsäurechloriden in Gegenwart von sauer wirkenden Katalysatoren.

Aus der EP-A-284 969 ist bekannt, Tetrahydropyran-4-carbonsäureester durch Umsetzung 3-(2'-Hydroxyethyl)-dihydro-2(3H)furanon oder dessen Estern mit Alkoholen in Gegenwart von sauer wirkenden Katalysatoren, herzustellen. Bei dieser Reaktion entstehen als Nebenprodukte 3-(2'-Alkoxyethyl)-dihydro-2(3H)furanone. Diese Ether lassen sich durch Rückführung ebenfalls zu Tetrahydropyran-4-carbonsäureester umsetzen. Allerdings liegen die Tetrahydropyran-4-carbonsäureester-Ausbeuten hierbei erheblich unter den Werten der oben beschriebenen Reaktion.

Weiterhin war bekannt, daß sich Ether mit Carbonsäuren oder Carbonsäureanhydriden an sauren Katalysatoren (z.B. in der DE-A-14 93 211 an Alumosilikaten; in der NL-A-67 07 444 an Schwefelsäure und in der US-A-4 734 521 an Sulfonsäuren auf Polymerträgern/Cu(I)CL) zu Carbonsäureestern umsetzen lassen.

Es war zu erwarten gewesen, daß unter den genannten drastischen Reaktionsbedingungen 3-(2'-Alkoxyethyl)-dihydro-2(3H)-furanone in zunehmendem Maße Eliminierungsprodukte als Nebenprodukte bilden.

Der vorliegenden Erfindung lag daher die Aufgabe zugrunde, den zuvor genannten Nachteilen abzuhelfen.

Demgemäß wurde ein neues und verbessertes Verfahren zur Herstellung von 3-(2'-Acyloxyethyl)-dihydro-2(3H)furanonen der allgemeinen Formel I 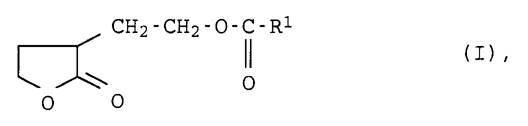 in der
- R¹: Wasserstoff, C₁- bis C₁₂-Alkyl, C₅- bis C₈-Cycloalkyl, Aryl, C₇- bis C₁₂-Aralkyl oder C₇- bis C₁₂-Alkylaryl
bedeutet, gefunden, welches dadurch gekennzeichnet ist, daß man 3-(2'-Oxyethyl)-dihydro-2(3H)furanone der allgemeinen Formel II 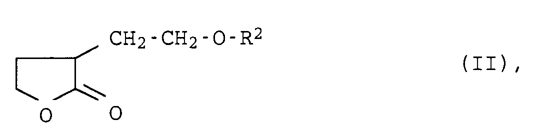 in der
- R²: C₁- bis C₁₂-Alkyl, C₅- bis C₈-Cycloalkyl, Aryl, C₇- bis C₁₂-Aralkyl oder C₇- bis C₁₂-Alkylaryl
bedeutet, mit Carbonsäuren (R¹-COOH), Carbonsäureanhydriden [(R¹CO)₂O] und/oder Carbonsäurehalogeniden (R¹-COHal) in Gegenwart von sauren Katalysatoren bei Temperaturen von 50 bis 250°C und Drücken von 0,1 bis 100 bar umsetzt.

Das erfindungsgemäße Verfahren läßt sich wie folgt durchführen:

Man kann die 3-(2'-Oxyethyl)-dihydro-2(3H)furanone II mit Carbonsäuren, Carbonsäureanhydriden und/oder Carbonsäurehalogeniden in Gegenwart von sauren Katalysatoren zusammengeben und bei erhöhten Temperaturen zur Reaktion bringen.

Die Umsetzung kann bei Temperaturen von 50 bis 250°C, vorzugsweise bei 100 bis 200°C und Drücken von 0,1 bis 100 bar, insbesondere von 1 bis 10 bar durchgeführt werden.

Die Reaktion kann kontinuierlich oder diskontinuierlich als Festbettreaktion mit Festbettkatalysatoren, beispielsweise nach der Sumpf- oder Rieselfahrweise oder aber mit in der Flüssigphase suspendierten Festbettkatalysatoren durchgeführt werden. Man kann aber auch in der Flüssigphase homogen gelöste saure Katalysatoren einsetzen.

Siedet der aus dem freigesetzten Alkohol und überschüssiger Carbonsäure gebildete Carbonsäureester niedriger als die eingesetzte Carbonsäure, so kann es sinnvoll sein, ihn, z.B. durch Destillation, kontinuierlich aus dem Reaktionsgemisch zu entfernen.

Geeignete 3-(2'-Oxyethyl)-dihydro-2(3H)furanone II, alles Ether, sind z.B. Methoxy-, Ethoxy-, Propoxy-, Butoxy- und tert.-Butoxy-ethyldihydro-2(3H)furanon.

Geeignete Carbonsäuren sind z.B. Ameisensäure, Essigsäure, Propionsäure, Buttersäure, Cyclohexancarbonsäure und Benzoesäure, geeignete Anhydride sind z.B. Acetanhydrid, Glutarsäureanhydrid und Benzoesäureanhydrid und geeignete Säurehalogenide sind z.B. Essigsäurechlorid, Propionsäurechlorid, Buttersäurechlorid und Benzoesäurechlorid.

Das Molverhältnis von Säure, Anhydrid bzw. Säurehalogenid zum Ether II beträgt vorteilhaft 1 : 1 bis 10 : 1, insbesondere 1,5 : 1.

Geeignete saure oder supersaure Katalysatoren sind z.B. Mineralsäuren wie Schwefelsäure oder Phosphorsäure, Sulfonsäuren wie Toluolsulfonsäure oder Fluorsulfonsäure, Zeolithe, z.B. mit Pentasilstruktur wie ZSM-5 oder ZSM-11, Alumosilikate wie Tonsil, Heteropolysäuren wie H₃PW₁₂O₄₀ oder Cs_{2,5}H_{0,5}PW₁₂O₄₀, Ionentauscher wie Perfluoralkylsulfonsäuren (Nafion) oder auf Polymerträgern gebundene Sulfonsäuren (z.B. Amberlyst), Lewissäuren wie Zinkchlorid oder Kupferchlorid und sauer wirkende Oxide wie Siliciumdioxid, Titandioxid, Zirkondioxid oder Aluminiumoxid, bevorzugt Mineralsäuren, Sulfonsäuren, Zeolithe, Ionentauscher und Heteropolysäuren, besonders bevorzugt Schwefelsäure, Zeolithe, Toluolsulfonsäure und Ionentauscher.

Die Substituenten R¹ und R² in den Verbindungen I und II haben folgende Bedeutungen:
- R¹, R²: - unabhängig voneinander
- C₁- bis C₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₅- bis C₈-Cycloalkyl wie Cyclopentyl, Cyclohexyl, Cycloheptyl und Cyclooctyl, bevorzugt Cyclopentyl, Cyclohexyl und Cyclooctyl, besonder bevorzugt Cyclopentyl und Cyclohexyl
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇- bis C₁₂-Aralkyl, bevorzugt C₇- bis C₁₂-Phenylalkyl wie Benzyl, 1-Phenethyl, 2-Phenethyl, 1-Phenyl-propyl, 2-Phenyl-propyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
- C₇- bis C₁₂-Alkylaryl, bevorzugt C₇- bis C₁₂-Alkylphenyl wie 2-Methylphenyl, 3-Methylphenyl, 4-Methylphenyl, 2,4-Dimethyl-phenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl,2-Ethylphenyl, 3-Ethylphenyl, 4-Ethylphenyl, 2-n-Propylphenyl, 3-n-Propylphenyl und 4-n-Propylphenyl.

Durch das erfindungsgemäße Verfahren läßt sich das wichtigste Nebenprodukt der Tetrahydropyran-4-carbonester - Synthese, das 3-(2'-Methoxyethyl)-dihydro-2(3H)furanon in das Edukt 3-(2'-Acetoxyethyl)-dihydro-2(3H)furanon umwandeln und damit in die Tetrahydropyran-4-carbonsäureester - Synthese zurückführen.

### Beispiele

### Beispiel 1

In einer Rührapparatur wird eine Mischung aus 20 g 3-(2'-Methoxyethyl)-dihydro-2(3H)furanon, 100 g Eisessig und 10 g Nafion auf 120°C erhitzt. Nach 69 Stunden wird die Reaktion abgebrochen (Umsatz: 89 %) und der Katalysator durch Filtration abgetrennt. Nach destillativer Aufarbeitung erhält man 65 % 3-(2'-Acetoxyethyl)-dihydro-2(3H)furanon (bezogen auf eingesetztes 3-(2'-Methoxyethyl)-dihydro-2(3H)furanon).

### Beispiel 2

In einer Rührapparatur wird eine Mischung aus 10 g 3-(2'-Methoxyethyl)-dihydro-2(3H)furanon, 10 g Eisessig und 1 g konz. Schwefelsäure auf 180°C erhitzt. Nach 19 Stunden wird die Reaktion abgebrochen. Der Reaktionsaustrag bestand laut GC-Analyse (Fl%) aus 86 % 3-(2'-Acetoxyethyl)-dihydro-2(3H)furanon und 11 % 3-(2'-Methoxyethyl)-dihydro-2(3H)-furanon.

### Beispiel 3

In einer Rührapparatur wird eine Mischung aus 72 g 3-(2'-Methoxyethyl)-dihydro-2(3H)furanon, 90 g Eisessig und 14 g Zeolith (ZSM-11) auf 150°C erhitzt. Nach 24 Stunden wird die Reaktion abgebrochen und der Katalysator durch Filtration abgetrennt. Der Austrag bestand laut GC-Analyse (Fl%) aus 75 % 3-(2'-Acetoxyethyl)-dihydro-2(3H)furanon, 9 % 3-(2'-Hydroxyethyl)-dihydro-2(3H)furanon und 9 % 3-(2'-Methoxyethyl)-dihydro-2(3H)furanon.

### Beispiel 4

In einer Rührapparatur wird eine Mischung aus 20 g 3-(2'-Methoxyethyl)-dihydro-2(3H)furanon, 100 g Acetanhydrid und 10 g Nafion auf 120°C erhitzt. Nach 69 Stunden wird die Reaktion abgebrochen (Umsatz: 100 %) und der Katalysator durch Filtration abgetrennt. Nach destillativer Aufarbeitung erhält man 61 % 3-(2'-Acetoxyethyl)-dihydro-2(3H)furanon.

### Beispiel 5

In einer Rührapparatur wird eine Mischung aus 10 g 3-(2'-tert-Butoxyethyl)-dihydro-2(3H)furanon, 50 g Eisessig und 5 g Nafion auf 120°C erhitzt. Nach 19 Stunden wird die Reaktion abgebrochen (Umsatz: 100 %) und der Katalysator durch Filtration abgetrennt. Nach destillativer Aufarbeitung erhält man 98 % 3-(2'-Acetoxyethyl)-dihydro-2(3H)furanon.

### Beispiel 6

In einer Rührapparatur wird eine Mischung aus 10 g 3-(2'-Methoxyethyl)-dihydro-2(3H)furanon, 20 g Propionsäure und 5 g Zeolith (ZSM-11) auf 150°C erhitzt. Nach 20 Stunden wird die Reaktion abgebrochen und der Katalysator durch Filtration abgetrennt. Der Austrag bestand laut GC-Analyse (Fl%) aus 84 % 3-(2'-Propionoxyethyl)-dihydro-2(3H)furanon, 7 % 3-(2'-Hydroxyethyl)-dihydro-2(3H)furanon und 7 % 3-(2'-Methoxyethyl)-dihydro-2(3H)furanon.

### Beispiel 7

In einer Rührapparatur wird eine Mischung aus 10 g 3-(2'-Methoxyethyl)-dihydro-2(3H)furanon, 40 g Cyclohexancarbonsäure und 5 g Zeolith (ZSM-11) auf 150°C erhitzt. Nach 25 Stunden wird die Reaktion abgebrochen und der Katalysator durch Filtration abgetrennt. Der Austrag bestand laut GC-Analyse (Fl%) aus 66 % Ester I, 4 % 3-(2'-Hydroxyethyl)-dihydro-2(3H)furanon und 11 % 3-(2'-Methoxyethyl)-dihydro-2(3H)furanon.

## Patentansprüche

1. Verfahren zur Herstellung von 3-(2'-Acyloxyethyl)-dihydro-2(3H)furanonen der allgemeinen Formel I 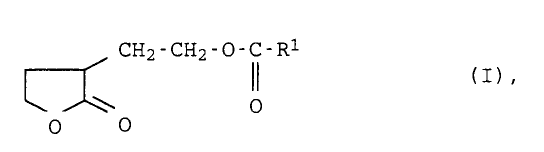 in der
R¹ Wasserstoff, C₁- bis C₁₂-Alkyl, C₅- bis C₈-Cycloalkyl, Aryl, C₇- bis C₁₂-Aralkyl oder C₇- bis C₁₂-Alkylaryl
bedeutet, dadurch gekennzeichnet, daß man 3-(2'-Oxyethyl)-dihydro-2(3H)furanone der allgemeinen Formel II 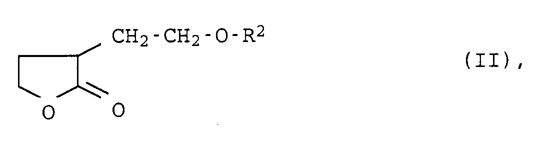 in der
R² C₁- bis C₁₂-Alkyl, C₅- bis C₈-Cycloalkyl, Aryl, C₇- bis C₁₂-Aralkyl oder C₇- bis C₁₂-Alkylaryl
bedeutet, mit Carbonsäuren (R¹-COOH), Carbonsäureanhydriden [(R¹CO)₂O] und/oder Carbonsäurehalogeniden (R¹-COHal) in Gegenwart von sauren Katalysatoren bei Temperaturen von 50 bis 250°C und Drücken von 0,1 bis 100 bar umsetzt.

2. Verfahren zur Herstellung von 3-(2'-Acyloxyethyl)-dihydro-2(3H)furanonen nach Anspruch 1, dadurch gekennzeichnet, daß man als saure Katalysatoren Mineralsäuren, Sulfonsäuren, Zeolithe, Alumosilikate, Heteropolysäuren, Ionentauscher, auf Polymerträgern gebundene Sulfonsäuren, Lewissäuren und/oder sauer wirkende Oxide einsetzt.

3. Verfahren zur Herstellung von 3-(2'-Acyloxyethyl)-dihydro-2(3H)furanonen nach Anspruch 2, dadurch gekennzeichnet, daß man als sauer wirkende Oxide Siliciumdioxid, Titandioxid, Zirkondioxid und/oder Aluminiumoxid einsetzt.

4. Verfahren zur Herstellung von 3-(2'-Acyloxyethyl)-dihydro-2(3H)furanonen nach Anspruch 1, dadurch gekennzeichnet, daß man die Umsetzung bei Temperaturen von 100 bis 200°C durchführt.

5. Verfahren zur Herstellung von 3-(2'-Acyloxyethyl)-dihydro-2(3H)furanonen nach Anspruch 1, dadurch gekennzeichnet, daß man die Umsetzung bei Drücken von 1 bis 10 bar durchführt.

## Claims

1. A process for preparing 3-(2'-acyloxyethyl)dihydro-2(3H)furanones of the formula I 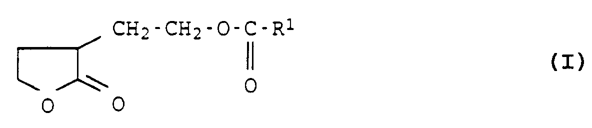 in which
R¹ is hydrogen, C₁- to C₁₂-alkyl, C₅- to C₈-cycloalkyl, aryl, C₇- to C₁₂-aralkyl or C₇- to C₁₂-alkylaryl,
which comprises reacting 3-(2'-oxyethyl)dihydro-2(3H)furanones of the formula II 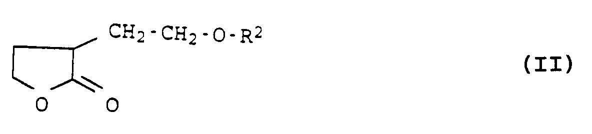 in which
R² is C₁- to C₁₂-alkyl, C₅- to C₈-cycloalkyl, aryl, C₇- to C₁₂-aralkyl or C₇- to C₁₂-alkylaryl
with carboxylic acids (R¹-COOH), carboxylic anhydrides [(R¹CO)₂O] and/or carbonyl halides (R¹-COHal) in the presence of acidic catalysts at temperatures from 50 to 250°C and pressures from 0.1 to 100 bar.

2. A process for preparing 3-(2'-acyloxyethyl)dihydro-2(3H)furanones as claimed in claim 1, wherein acidic catalysts employed are mineral acids, sulfonic acids, zeolites, alumosilicates, heteropoly acids, ion exchangers, sulfonic acids bound to polymer supports, Lewis acids and/or acidic oxides.

3. A process for preparing 3-(2'-acyloxyethyl)dihydro-2(3H)furanones as claimed in claim 2, wherein acidic oxides employed are silicon dioxide, titanium dioxide, zirconium dioxide and/or aluminum oxide.

4. A process for preparing 3-(2'-acyloxyethyl)dihydro-2(3H)furanones as claimed in claim 1, wherein the reaction is carried out at temperatures from 100 to 200°C.

5. A process for preparing 3-(2'-acyloxyethyl)dihydro-2(3H)furanones as claimed in claim 1, wherein the reaction is carried out at pressures from 1 to 10 bar.

## Revendications

1. Procédé de fabrication de 3-(2'-acyloxyéthyl)-dihydro-2(3H)furanones de formule générale I 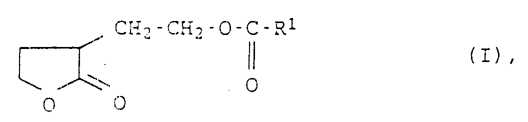 dans laquelle
R¹ représente un atome d'hydrogène, un groupement alkyle en C₁-C₁₂, cycloalkyle en C₅-C₈, aryle, aralkyle en C₇-C₁₂ ou alkylaryle en C₇-C₁₂, caractérisé en ce que l'on fait réagir des 3-(2'-oxyéthyl)-dihydro-2(3H)furanones de formule générale II 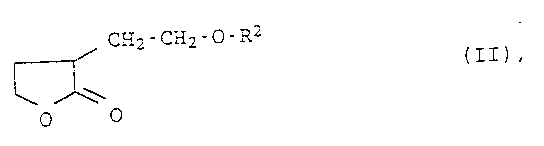 dans laquelle
R² représente un groupement alkyle en C₁-C₁₂, cycloalkyle en C₅-C₈, aryle, aralkyle en C₇-C₁₂ ou alkylaryle en C₇-C₁₂, avec des acides carboxyliques (R¹-COOH), des anhydrides d'acide carboxyliques [(R¹CO)₂O] et/ou des halogénures d'acide carboxylique (R¹-COHal) en présence de catalyseurs acides à des températures de 50 à 250°C et à des pressions de 0,1 à 100 bar.

2. Procédé de fabrication de 3-(2'-acyloxyéthyl)-dihydro-2(3H)furanones selon la revendication 1, caractérisé en ce que l'on utilise en tant que catalyseurs acides des acides minéraux, des acides sulfoniques, des zéolithes, des silicates d'aluminium, des hétéropolyacides, un échangeur d'ions, des acides sulfoniques fixés sur des supports polymères, des acides de Lewis et/ou des oxydes à action acide.

3. Procédé de fabrication de 3-(2'-acyloxyéthyl)-dihydro-2(3H)furanones selon la revendication 2, caractérisé en ce que l'on utilise en tant qu'oxydes à action acide du dioxyde de silicium, du dioxyde de titane, du dioxyde de zirconium, et/ou de l'oxyde d'aluminium.

4. Procédé de fabrication de 3-(2'-acyloxyéthyl)-dihydro-2(3H)furanones selon la revendication 1, caractérisé en ce que l'on mène la réaction à des températures de 100 à 200°C.

5. Procédé de fabrication de 3-(2'-acyloxyétnyl)-dihydro-2(3H)furanones selon la revendication 1, caractérisé en ce que l'on mène la réaction à des pressions de 1 à 10 bar.
